# EUROPÄISCHE PATENTSCHRIFT

(11) **EP 0 368 131 B1**
(45) Veröffentlichungstag und Bekanntmachung des Hinweises auf die Patenterteilung: **04.09.1996**
(21) Anmeldenummer: 89120245.9
(22) Anmeldetag: 02.11.1989
(51) Int. Cl.: C07K 14/435

(54) **Neoglykoproteine, ihre Herstellung und Verwendung**
Neoglucoproteins, their preparation and use
Néoglycoprotéines, leur préparation et utilisation

(30) Priorität: 05.11.1988 DE 3837623
(43) Veröffentlichungstag der Anmeldung: 16.05.1990
(73) Patentinhaber: BEHRINGWERKE Aktiengesellschaft, 35001 Marburg (DE)
(72) Erfinder: Wiegand, Herbert, Prof.Dr., D-3550 Marburg (DE); Bosslet, Silke, D-3550 Marburg (DE); Bosslet, Klaus, Dr., D-3550 Marburg (DE); Sedlacek, Klaus, Dr., D-3550 Marburg (DE)

(56) Entgegenhaltungen:
- US-A- 4 557 931
- SPDP HETEROBIFUNCTIONAL REAGENT PHARMACIA FINE CHEMICALS, 1978, Uppsala (SE); Zeilen 1-12
- JOURNAL OF HISTOCHEMISTRY & CYTOCHEMISTRY, Band 32, Nr. 10, 1984, The Histochemical Society Inc., New Brunswick, NJ (US); M. KATAOKA et al., Seiten 1091-1098

## Beschreibung

Die Erfindung betrifft die Synthese und Verwendung von Neoglykoproteinen. Monosialolaktose und Disialolaktose werden aus Kuh-Kolostrum isoliert und mittels eines Kopplungsreagenzes an humanes Serumalbumin (HSA) als Trägerprotein gekoppelt. Diese Neoglykoproteine sind als Vakzine, daneben auch als Liganden zur Lokalisation und Therapie von lektin-exprimierenden Tumorgeweben geeignet.

Ganglioside, d.h. sialinsäurehaltige Glykosphingolipide, sind Plasmamembranlipide, die aus einem hydrophoben Ceramidanteil und einem hydrophilen Kohlenhydratanteil bestehen. Mit dem Ceramidanteil, der aus Sphingosin und einer langkettigen Fettsäure zusammengesetzt ist, sind Glykosphingolipide so in der äußeren Plasmamembran verankert, daß die Oligosaccharidketten in den Extrazellulärraum ragen. Aufgrund der Tatsache, daß sie auf der Zelloberfläche zelldifferenzierungs- und speziesspezifische Muster bilden, nimmt man an, daß ihnen im Zellgeschehen eine biologisch wichtige Funktion zukommt.

Glykosphingolipide sind typisch für alle Säugetierzellen, kommen aber meist nur in geringen Mengen vor. Besonders angereichert sind sialinsäurehaltige Glykosphingolipide, d.h. Ganglioside, auf neuronalen Plasmamembranen. Auch auf den Zelloberflächen von Tumoren neuroektodermalen Ursprungs (wie Melanoma, Astrocytoma, Neuroblastoma etc.) werden Ganglioside stark exprimiert. Dabei handelt es sich zum Teil um Ganglioside, die auf normalen Säugetierzellen, aber auch im Gehirn und auf Nervenfasern nur in relativ geringen Mengen vorkommen. Aus diesem Grund sind die betroffenen Ganglioside GM2, GM3, GD2 und GD3 als tumor-assoziierte Antigene interessant geworden. Auch die Möglichkeiten einer Tumorimmuntherapie von Tumoren neuroektodermalen Ursprungs mit diesen Gangliosiden als Zielantigen werden von verschiedenen Arbeitspruppen untersucht. Zwei unterschiedliche Ansätze werden bisher zum Teil erfolgreich bearbeitet:
1. Anwendung monoklonaler Antikörper, die gegen die entsprechenden Ganglioside gerichtet sind und
2. aktive Immunisierung mit den entsprechenden Gangliosiden.

Untersuchungen haben gezeigt, daß Antikörper gegen gereinigtes GM2-Gangliosid in Melanom-Patienten nicht hervorgerufen werden können. Erst die Anwendung von Adjuvanzien wie Bacillus Calmette-Guérin (BCG) oder Salmonella minnesota, sowie eine Vorbehandlung der Patienten mit Cyclophosphamid induzieren IgM-Antikörper gegen das Gangliosid GM2 (Livingston et al. (1987), Proc. Natl. Acad. Sci. 84, 2911-2915). Auch eine Ganzzellvakzine, bestehend aus humanen oder murinen Melanom- bzw. Astrocytomzellinien, die einen hohen GM2-Gangliosid-Gehalt aufweisen, wurden Melanom-Patienten injiziert und führten zu einer Induktion von Anti-GM2-Antikörper des IgM-Isotops (Livingston et al. a.a.O.).

Die bisherigen Studien an Melanom-Patienten zeigen, daß ein erhöhter Antikörper-Titer gegen das Gangliosid GM2 mit einer verlängerten Überlebenszeit korreliert.

Zentrale Probleme bei der Konstruktion einer Gangliosid-Vakzine sind die schlechte Immunogenität der gereinigten Ganglioside sowie ihre schwierige Isolierung.

Solche Schwierigkeiten bestehen generell auch bei der Untersuchung zuckerbindender Proteine. Deshalb wurden beispielsweise Neoglykoproteine entwickelt, enthaltend einen Einfachzucker, wie z. B. Galaktose und ein Trägerprotein, wie z. B. Rinderserumalbumin (BSA), um die Bindung von Lektinen besser analysieren zu können [J. Histochem. and Cytochem. (1984), 32 (10), pp. 1091-1098].

Ein Ansatz, die Immungenität des Gangliosids GM2 zu erhöhen, findet sich in der U.S`-Patentschrift Nr. 4,557,931. Dort wird ein Neoglykoprotein offenbart, dessen Zuckeranteil von GM2 stammt und dessen Proteinanteil HSA ist. Dieses Oligosaccharid-HSA-Konjugat wird als Vakzine zur Induktion von Antikörpern, die mit GM2 reagieren sowie als diagnostisches Mittel verwendet.

Es war ferner bekannt, daß die Zuckeranteile der Ganglioside GM3 und GD3, Monosialolaktose bzw. Disialolaktose, aus Kuh-Kolostrum leicht zugänglich sind. Wir haben überraschenderweise gefunden, daß Monosialolaktose bzw. Disialolaktose, mittels eines Kopplungsreagenz an HSA gekoppelt, mit monoklonalen Antikörpern reagieren, die gegen die Ganglioside GM3 bzw. GD3 erzeugt wurden. Umgekehrt sind die oben genannten Neoglykoproteine in der Lage, Antikörper zu erzeugen, die mit den Gangkiosiden GM3 bzw. GD3 reagieren und sind daher als Vakzine zur spezifischen Tumorimmuntherapie geeignet. Des weiteren sind sie auch z. B. als Liganden zur Lokalisation und Therapie von lektinexprimierenden Tumorgeweben geeignet.

Die Erfindung betrifft folglich
a) Neoglykoproteine, zusammengesetzt aus Monosialo- oder Disialolaktose, mit Trägerproteinen, darunter auch Enzyme, bevorzugt humanes Serumalbumin,
b) Verfahren zu ihrer Herstellung, wobei bevorzugt von Kuh-Kolostrum als Zuckerquelle ausgegangen wird.
c) sowie ihre Verwendung als Vakzine oder als Diagnosehilfsmittel, z. B. bei Nachweis von Gangliosiden in kompetitiven Radioimmunoassays im Rahmen der Tumordiagnostik sowie als Therapeutikum bzw. Diagnostikum bei lektinexprimierenden Tumoren.

Die Erfindung ist ferner in den Beispielen und den Patentansprüchen enthalten.

### Beispiele

### 1. Gewinnung von Monosialolaktose und Disialolaktose aus Kuh-Kolostrum

Kuh-Kolostrum wurde mit geringen Modifikationen nach der - Methode von v. Nicolai aufgearbeitet (v. Nicolai, Dissertation Universität Bonn, 1971).

Kuh-Kolostrum (10 Liter) wurde durch Zentrifugation entfettet und Proteine mit 50% Aceton in der Kälte gefällt. Nach erneuter Zentrifugation wurde der Überstand am Rotationsverdampfer auf 1/10 des Originalvolumens eingedampft.

Die Rohfraktion wurde über eine Sephadex G-15-Säule entsalzt und die Zucker-positiven sowie Chlorid-negativen Fraktionen vereinigt und eingeengt. Die Trennung der einzelnen Zucker wurde durch Ionenaustausch über eine DEAE-Sephadex A-25-Säule in 0.1 M Tris/HCl-Puffer, pH 7.5, mit einem Natriumchlorid-Gradienten erreicht. Die entsprechenden Zuckerfraktionen wurden vereinigt, eingeengt und über eine Sephadex G-15-Säule entsalzt.

Der Zuckernachweis in den einzelnen Fraktionen der Säulenaufarbeitungsschritte erfolgte mit dem Ehrlich- und dem Anthron-Test bzw. durch dünnschichtchromatographische Auftrennung auf Kieselgelplatten im Laufmittelgemisch Pyridin/Ethylacetat/Eisessig/Wasser (6:3:1:3) und Ansprühen der Platte mit Resorcin.

### 2. Kopplung von Monosialolaktose und Disialolaktose auf humanes Serumalbumin (HSA) mittels des Kopplungsreagenzes SPDP

Für die Kopplung der Zucker an Protein gibt es prinzipiell mehrere Möglichkeiten. Die Zucker können z.B. direkt an die freien epsilon-Aminolysyl-Gruppen des Proteins gekoppelt werden, oder aber auch über homo- oder heterobifunktionelle Kopplungsreagenzien.

Im nachfolgenden sind die Kopplungsreaktionen unter Verwendung von N-Succinimidyl-3-(2-pyridyldithio)propionat (SPDP) als Kopplungsreagenz beschrieben. Die Reaktionsschritte entsprechen weitgehend der veröffentlichten Methode (J. Carlsson et al.(1978), Biochem. J. 173, 723-737).

### a) Reduktaminierung der Monosialolaktose bzw. Disialolaktose

Nach der Methode von Wiegandt wurde Monosialolaktose bzw. Disialolaktose in methanolischer Ammoniumacetat-Lösung unter Zusatz von Natriumcyanoborhydrid am C₁-Atom der Glukose reduktaminiert (H.Wiegandt, W.Ziegler, (1974) Hoppe-Seyler's Z. Physiol. Chem. 355, 11-18).

Das reduktaminierte Zucker-Derivat wurde über eine Biogel P2-Säule gereinigt.

Im folgenden sind R₁ und R₂ wie folgt definiert: 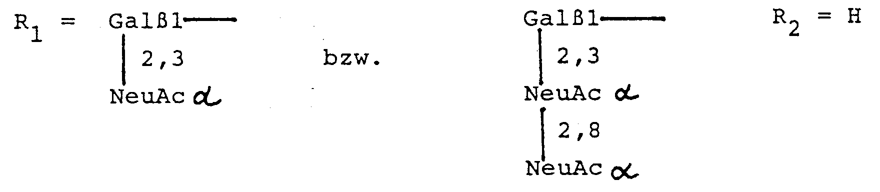
- Abkürzungen:: Gal: Galaktose
NeuAc: N-Acetylneuraminsäure

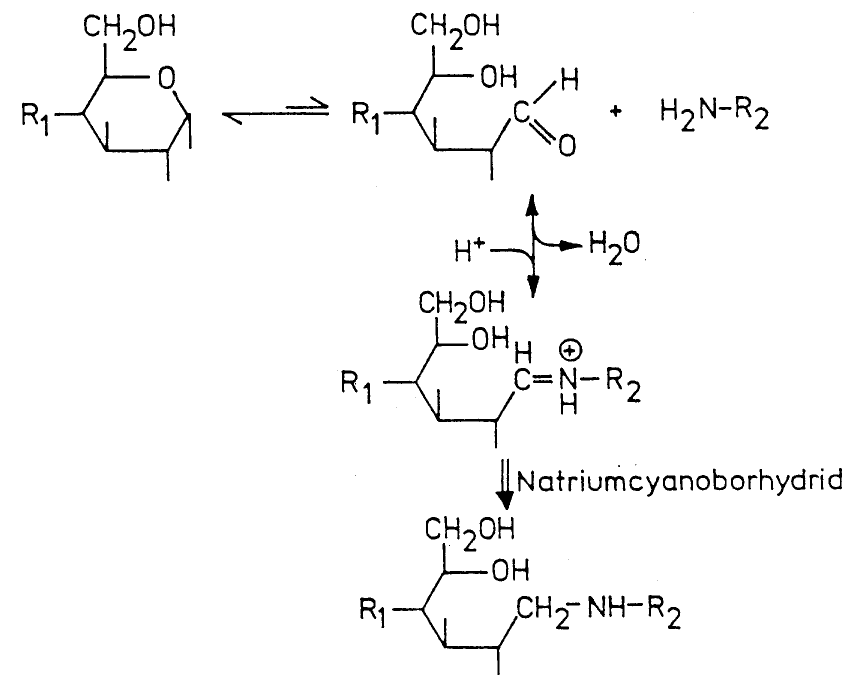

### b) Umsetzung des reduktaminierten Zuckers mit SPDP

Die Derivatisierung wurde in 0.1 M Natriumphosphat-Puffer + 0.1 M Natriumchlorid, pH 7.5 mit 3-5fachem molaren SPDP Überschuß durchgeführt. Die Reaktionszeit bei Raumtemperatur betrug 2-12 Stunden.

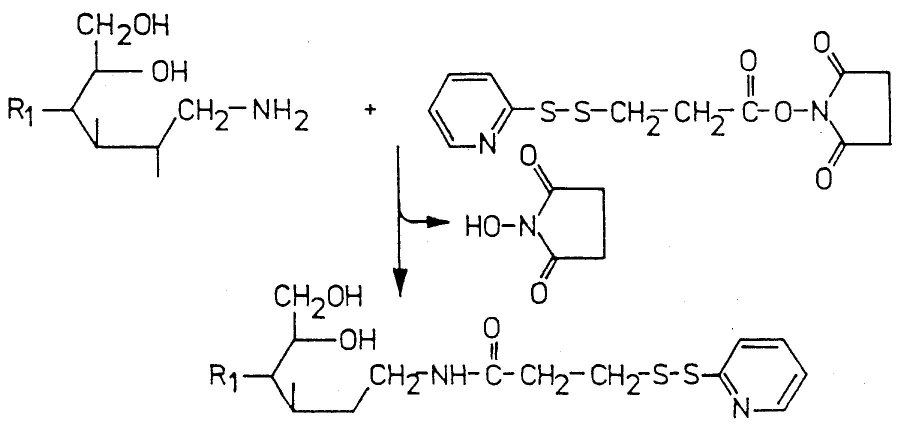

Das Zucker-NH₂-SPDP-Derivat wurde von N-Hydroxysuccinimid und unreagiertem SPDP über eine Biogel P2-Säule abgetrennt. Bei auftretender SPDP-Verunreinigung des Derivates wurde ein zusätzlicher Reinigungschritt über ein Kieselgelsäulchen gemacht, das mit Lösungsmittelgemischen steigender Polarität eluiert wurde.

### c) Umsetzung von HSA mit SPDP

Die Proteinderivatisierung erfolgte unter den gleichen Bedingungen wie die Zuckerderivatisierung in 0.1 M Natriumphosphat-Puffer, pH 7.5, mit 3-5fachem molaren, auf freie epsilon-Aminolysylgruppen bezogenen SPDP-Überschuß.

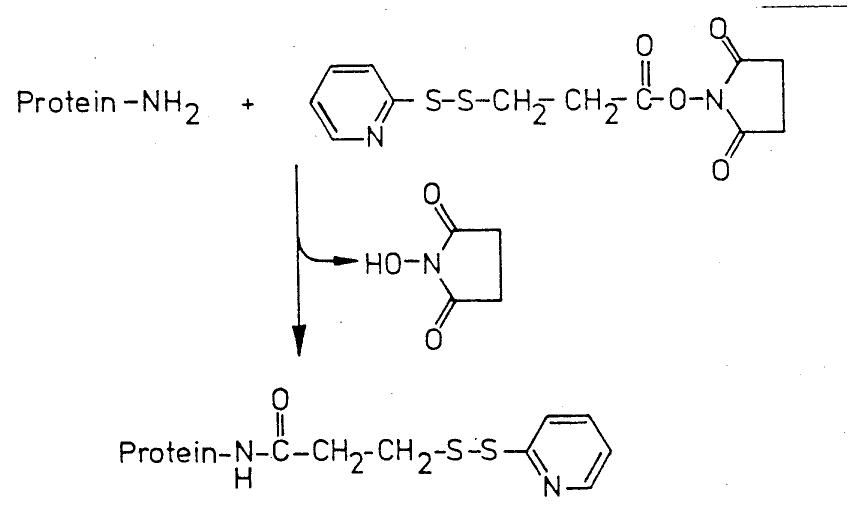

Die Abtrennung des Protein-SPDP-Derivates erfolgte über eine Sephadex G-25-Säule, die mit dem Puffer für die nachfolgenden Reaktionen (0.1 M Natriumphosphat-Puffer, pH 6, 5 mM EDTA) eluiert wurde.

### d) Reduktion des HSA-SPDP-Derivates

Unter Zusatz von 25 mM Dithiothreitol wurden die durch die Derivatisierung neu eingeführten Disulfidbrücken des HSA-SPDP-Derivates in 0.1 M Natriumphosphat-Puffer, pH 6, 5 mM EDTA unter Abspaltung von 2-Thiopyridon reduziert. Unter diesen Reaktionsbedingungen werden die nativen Disulfidbrücken des Proteins nicht reduziert. Reaktion erfolgte bei Raumtemperatur und die Reaktionszeit betrug 1-2 Stunden.

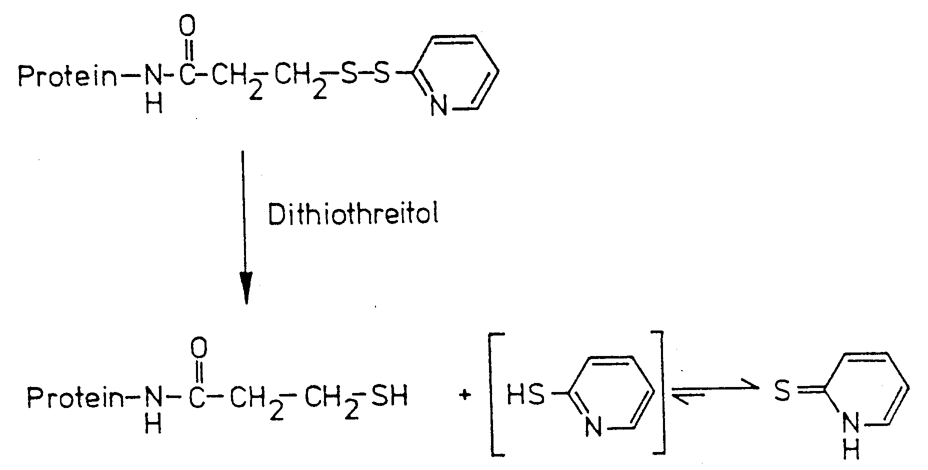

Die Abtrennung des reduzierten HSA-SPDP-Derivates erfolgte über eine Sephadex G-25-Säule mit 0.1 M Natriumphosphat-Puffer, pH 6, 5 mM EDTA als Elutionspuffer.

### e) Kopplung des Zucker- und des Protein-Derivates

Reduziertes HSA-SPDP-Derivat wurde sofort mit dem Zucker-NH₂-SPDP-Derivat umgesetzt: Das Zucker-NH₂-SPDP-Derivat wurde in 2-5fachem molaren Überschuß, bezogen auf epsilon-Aminolysylgruppen des Proteins, eingesetzt, die Reaktionszeit betrug 24-48 Stunden bei Raumtemperatur.

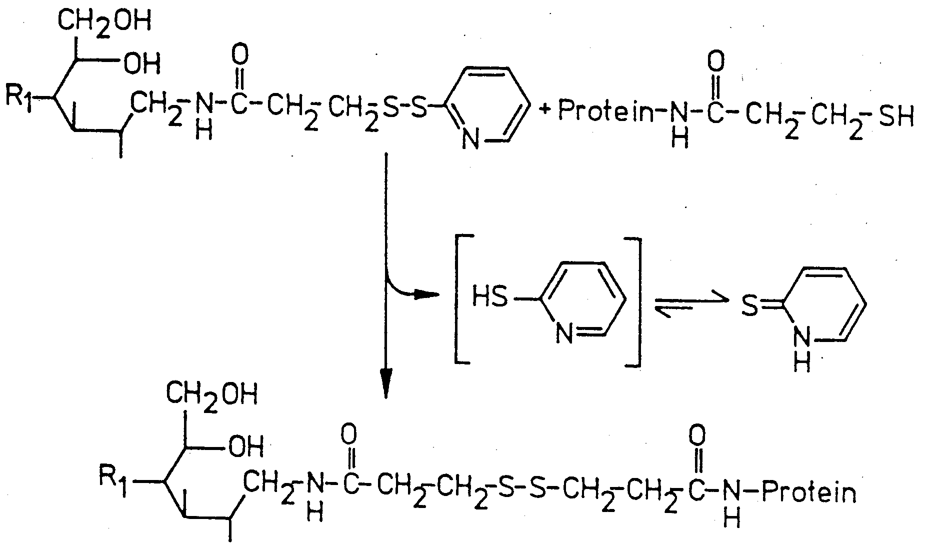

Das Kopplungsprodukt bzw. das Neoglykoprotein wurde durch eine Säulenchromatographie über Sephadex G-25 von den Reaktionsprodukten abgetrennt.

In der beschriebenen Prozedur werden reduktaminierte Zucker über SPDP an freie epsilon-Aminolysylgruppen des Proteins gekoppelt. Alternativ können auch die Disulfidbrücken des Proteins reduziert werden und der reduktaminierte Zucker über SPDP an die nun freien Sulfhydrylgruppen der Cystein-Reste gekoppelt werden.

### 3. Nachweismethoden zur Identifikation der Zwischenprodukte

Folgende Nachweismethoden wurden wahlweise zur Identifizierung der Zwischenprodukte eingesetzt:
A) für Monosialolaktose bzw. Disialolaktose und die entsprechenden Derivate
   a) verändertes dünnschichtchromatographisches Laufverhalten nach Reduktaminierung und SPDP-Derivatisierung auf Kieselgel G-60 Platten in den Laufmitteln:
      Pyridin / Ethylacetat / Eisessig / Wasser (6:3:1:3) Chloroform / Methanol / 0.2% wäßriges Kalziumchlorid (60:35:8) und Anfärbbarkeit der Banden mit Joddampf, Ninhydrin und Resorcin.
   b) Quantifizierung der SPDP-Derivatisierung durch leicht modifizierte Neuraminsäurebestimmung mit 1,2-Diamino-4,5-dimethoxybenzol, (S. Hara et al. (1986) J.Chromatography 377, 111-119) und durch Abspaltung von 2-Thiopyridon mit Dithiothreitol. Die Menge an freigewordenen 2-Thiopyridon, das der Menge an SPDP-derivatisiertem Zucker entspricht, kann durch Messung der Extinktionszunahme bei der Wellenlänge von lambda = 343 nm im Photometer gemessen werden und die Konzentration mit Hilfe des Lambert-Beer-Gesetzes errechnet werden. Der molare Extinktionskoeffizient für 2-Thiopyridon bei lambda = 343 nm beträgt 7.06 x 10³ x M⁻¹x cm⁻¹ (D.R.Grassetti & J.F.Murray, (1967) Arch. Biochem. Biophys. 119, 41-49).
   c) GC-Analyse der einzelnen Zucker-Derivate nach Reduktaminierung und SPDP-Derivatisierung nach J. Lechner et al. (1985) J. Biol. Chem. 260, 860-866.
B) für HSA
   a) Proteinbestimmung wurde mit dem Bio-Rad Protein Assay durchgeführt.
   b) die SPDP-Derivatisierung wurde quantifiziert durch Proteinbestimmung mit dem Bio-Rad Protein Assay und durch Abspaltung von 2-Thiopyridon mit Dithiothreitol. Freigewordenes 2-Thiopyridon, das der Menge an gebundenem SPDP entspricht, wurde durch die Extinktionszunahme bei der Wellenlänge lambda = 343 nm im Photometer gemessen und die Konzentration mit Hilfe des Lambert-Beer-Gesetzes errechnet. Der molare Extinktionskoeffizient für 2-Thiopyridon bei lambda = 343 nm beträgt 7.06 x 10³ x M⁻¹ x cm⁻¹ (Grassetti & Murray, a.a.O.).
   c) HSA und HSA-SPDP-Derivate zeigen in der SDS-Gel-elektrophorese, angefärbt mit Coomassie-Blue, unterschiedliches Laufverhalten.
4. Synthese von Disialolaktose-HSA-Konjugat

In einem charakteristischen Versuchsansatz wurden 10 mg (10.75 µmol) Disialolaktose in 2 ml einer methanolischen Ammoniumacetat-Lösung unter Zusatz von 20 mg Natriumcyanoborhydrid reduktaminiert. Der über eine Biogel-P2-Säule gereinigte, reduktaminierte Zucker wurde anschliessend mit 13 mg SPDP, gelöst in 1050 µl Ethanol, in 13 ml 0.1 M Natriumphosphat-Puffer, pH 7.5, umgesetzt und das Disialolaktose-NH₂-SPDP-Derivat über eine Biogel-P2-Säule und eine Kieselgelsäule gereinigt. Die Ausbeute, gemessen durch Neuraminsäurebestimmung mit 1,2-Diamino-4,5-dimethoxybenzol und durch Abspaltung von 2-Thiopyridon mit Dithiothreitol betrug ca. 50% (4.8-5.2 µmol) der eingesetzten Menge an Disialolaktose.

Für die Derivatisierung von HSA wurden 5.6 mg SPDP in 450 µl Ethanol gelöst und zu 6 mg HSA in 5550 µl 0.1 M Natriumphosphat-Puffer, pH 7.5, gegeben. Nach der Reinigung über eine Sephadex G-25-Säule erhält man ca. 4.8 mg HSA-SPDP-Derivat mit einer Derivatisierung von 45-53 SPDP-Molekülen pro HSA-Molekül.

Die neu eingeführten Disulfidbrücken im HSA-SPDP-Derivat wurden in 0.1 M Natriumphosphat-Puffer + 5 mM EDTA, pH 6, mit 25 mM Dithiothreitol gespalten. Die Reaktionszeit betrug bei Raumtemperatur 1 Stunde. Nach der Reinigung über eine Sephadex G-25-Säule wurde das reduzierte HSA-SPDP-Derivat sofort mit dem Disialolaktose-NH₂-SPDP-Derivat in 0.1 M Natriumphosphat-Puffer + 5 mM EDTA, pH 6, umgesetzt. Die Kopplungsreaktion konnte im Photometer durch eine Extinktionszunahme bei der Wellenlänge lambda = 343 nm, bedingt durch die Abspaltung von 2-Thiopyridon, verfolgt werden. Nach 24-48 Stunden erfolgte keine Extinktionszunahme mehr, die Kopplungsreaktion war beendet.

Nach Reinigung des Kopplungsproduktes über eine Sephadex G-25-Säule erhielt man 2.1-2.5 mg Disialolaktose-HSA-Konjugat, das mit 30-33 Disialolaktose-Molekülen pro HSA-Molekül derivatisiert war.

Der Derivatisierungsgrad des Kopplungsproduktes wurde bestimmt durch 1.) Proteinbestimmung mit dem Bio-Rad Protein Assay und 2.a) durch Neuraminsäurebestimmung des Kopplungsproduktes mit 1,2-Diamino-4,5-dimethoxybenzol, b) durch Berechnung der Konzentration an freigewordenem 2-Thiopyridon (beim letzten Kopplungschritt) durch Extinktionsmessung bei der Wellenlänge lambda = 343 nm und Einsetzen der entsprechenden Parameter in die Lambert-Beer-Gleichung und c) durch Bestimmung der Konzentration an wiedergewonnenem, nicht-reagierten Disialolaktose-NH₂-SPDP-Derivat mit den unter a) und b) genannten Methoden.

### 5. Immunologische Reaktionen des Disialolaktose-HSA-Konjugats

Das Kopplungsprodukt (Disialolaktose-HSA-Konjugat) wurde anschließend im Bio-Dot-Assay mit monoklonalen Anti-GD3-Antikörpern getestet: Sowohl mab 704/16 als mab 624/253 (beide gegen das Gangliosid GD3 gerichtet) reagierten mit dem Neoglykoprotein.

Auch nach SDS-Gelelektrophorese unter nicht-reduzierenden Bedingungen und anschließendem Western-Blot konnte die Reaktion der monoklonalen Antikörper mab 704/16 und mab 625/253 mit dem Disialolaktose-HSA-Konjugat gezeigt werden.

Immunisierungsversuche in Mäusen haben gezeigt, daß das Disialolaktose-HSA-Konjugat wesentlich immunogener als das Gangliosid GD3 ist, d.h. eine stärkere humorale Immunantwort hervorruft.

## Patentansprüche (Patentansprüche für folgende(n) Vertragsstaat(en): AT, BE, CH, DE, FR, GB, IT, LI, LU, NL, SE)

1. Neoglykoprotein, bestehend aus einem Trägerprotein und dem Zucker Mono- oder Disialolaktose.

2. Neoglykoprotein nach Anspruch 1, dadurch gekennzeichnet, daß das Trägerprotein humanes Serumalbumin (HSA) ist.

3. Verfahren zur Herstellung eines Neoglykoproteins nach Anspruch 1, dadurch gekennzeichnet, daß der Zucker an das Trägerprotein gekoppelt wird.

4. Verfahren zur Herstellung eines Neoglykoproteins nach Anspruch 1, dadurch gekennzeichnet, daß der Zucker an das Trägerprotein kovalent gekoppelt wird.

5. Verfahren zur Herstellung eines Neoglykoproteins nach Anspruch 4, dadurch gekennzeichnet, daS N-Succinimidyl-3-(2-pyridyldithio)propionat (SPDP) als Kopplungsreagenz eingesetzt wird.

6. Verfahren zur Herstellung eines Neoglykoproteins nach Anspruch 3, dadurch gekennzeichnet, daß als Zuckerquelle - Kuh-Kolostrum eingesetzt wird.

7. Verwendung eines Neoglykoproteins nach Anspruch 1 oder erhältlich nach Anspruch 3 zur Herstellung einer Vakzine.

8. Verwendung eines Neoglykoproteins nach Anspruch 1 oder erhältlich nach Anspruch 3 zur Herstellung eines diagnostischen Mittels.

9. Verwendung eines Neoglykoproteins nach Anspruch 1 in einem kompetitiven Radioimmunoassay zum Nachweis von Gangliosiden.

10. Vakzine, die ein Neoglykoprotein nach Anspruch 1 enthält.

## Patentansprüche (Patentansprüche für folgende(n) Vertragsstaat(en): ES, GR)

1. Verfahren zur Herstellung eines Neoglykoproteins, dadurch gekennzeichnet, daß der Zucker Mono- oder Disialolaktose mittels eines Kopplungsreagenzes an ein Trägerprotein gekoppelt wird.

2. Verfahren nach Anspruch 1, dadurch gekennzeichnet, daß das Kopplungsreagenz eine kovalente Bindung bewirkt.

3. Verfahren nach Anspruch 1, dadurch gekennzeichnet, daß als Trägerprotein humanes Serumalbumin (HSA) eingesetzt wird.

4. Verfahren nach Anspruch 1, dadurch gekennzeichnet, daß als Zuckerquelle Kuh-Kolostrum eingesetzt wird.

5. Verfahren nach Anspruch 2, dadurch gekennzeichnet, daß N-Succinimidyl-3-(2-pyridyldithio)propionat (SPDP) als Kopplungsreagenz eingesetzt wird.

6. Verfahren zur Herstellung einer Vakzine, dadurch gekennzeichnet, daß nach Anspruch 1 erhältliche Neoglykoproteine mit handelsüblichen Trägern und Stabilisatoren gemischt werden.

7. Verfahren zur Herstellung eines diagnostischen Mittels, dadurch gekennzeichnet, daß nach Anspruch 1 erhältliche Neoglykoproteine zum Einsatz kommen.

## Claims (Claims for the following Contracting State(s): AT, BE, CH, DE, FR, GB, IT, LI, LU, NL, SE)

1. A neoglycoprotein consisting of a carrier protein and the sugar mono- or disialolactose.

2. A neoglycoprotein as claimed in claim 1, wherein the carrier protein is human serum albumin (HSA).

3. A process for the preparation of a neoglycoprotein as claimed in claim 1, which comprises coupling the sugar to the carrier protein.

4. The process for the preparation of a neoglycoprotein as claimed in claim 1, which comprises covalently coupling the sugar to the carrier protein.

5. The process for the preparation of a neoglycoprotein as claimed in claim 4, wherein N-succinimidyl 3-(2-pyridyldithio)propionate (SPDP) is used as coupling reagent.

6. The process for the preparation of a neoglycoprotein as claimed in claim 3, wherein bovine colostrum is used as source of sugar.

7. The use of a neoglycoprotein as claimed in claim 1 or obtainable as claimed in claim 3 for the production of a vaccine.

8. The use of a neoglycoprotein as claimed in claim 1 or obtainable as claimed in claim 3 for the production of a diagnostic agent.

9. The use of a neoglycoprotein as claimed in claim 1 in a competitive radioimmunoassay for detecting gangliosides.

10. A vaccine which contains a neoglycoprotein as claimed in claim 1.

## Claims (Claims for the following Contracting State(s): ES, GR :)

1. A process for the preparation of a neoglycoprotein, which comprises coupling the sugar mono- or disialolactose to a carrier protein by means of a coupling reagent.

2. The process as claimed in claim 1, wherein the coupling reagent results in a covalent bond.

3. The process as claimed in claim 1, wherein human serum albumin (HSA) is used as carrier protein.

4. The process as claimed in claim 1, wherein bovine colostrum is used as source of sugar.

5. The process as claimed in claim 2, wherein N-succinimidyl 3-(2-pyridyldithio)propionate (SPDP) is used as coupling reagent.

6. A process for the production of a vaccine, which comprises mixing neoglycoproteins obtainable as claimed in claim 1 with conventional carriers and stabilizers.

7. A process for the production of a diagnostic agent, which comprises using neoglycoproteins obtainable as claimed in claim 1.

## Revendications (Revendications pour l'(les) Etat(s) contractant(s) suivant(s): AT, BE, CH, DE, FR, GB, IT, LI, LU, NL, SE)

1. Néoglycoprotéine constituée d'une protéine porteuse et du sucre mono- ou disialolactose.

2. Néoglycoprotéine selon la revendication 1, caractérisée en ce que la protéine porteuse est l'albumine de sérum humain (ASH).

3. Procédé pour la préparation d'une néoglycoprotéine selon la revendication 1, caractérisé en ce que le sucre est couplé à la protéine porteuse.

4. Procédé pour la préparation d'une néoglycoprotéine selon la revendication 1, caractérisé en ce que le sucre est couplé à la protéine porteuse par covalence.

5. Procédé pour la préparation d'une néoglycoprotéine selon la revendication 4, caractérisé en ce que l'on utilise comme réactif de couplage le 3-(2-pyridyldithio)propionate de N-succinimidyle (SPDP).

6. Procédé pour la préparation d'une néoglycoprotéine selon la revendication 3, caractérisé en ce que l'on utilise comme source de sucre le colostrum de vache.

7. Utilisation d'une néoglycoprotéine selon la revendication 1 ou pouvant être obtenue selon la revendication 3, pour la fabrication d'un vaccin.

8. Utilisation d'une néoglycoprotéine selon la revendication 1 ou pouvant être obtenue selon la revendication 3, pour la fabrication d'un agent de diagnostic.

9. Utilisation d'une néoglycoprotéine selon la revendication 1, dans un essai radio-immunologique compétitif destine à la détection de gangliosides.

10. Vaccin contenant une néoglycoprotéine selon la revendication 1.

## Revendications (Revendications pour l'(les) Etat(s) contractant(s) suivant(s): ES, GR)

1. Procédé pour la préparation d'une néoglycoprotéine, caractérisé en ce que le sucre mono- ou disialolactose est couplé à une protéine porteuse au moyen d'un réactif de couplage.

2. Procédé selon la revendication 1, caractérisé en ce que le réactif de couplage produit une liaison covalente.

3. Procédé selon la revendication 1, caractérisé en ce que l'on utilise comme protéine porteuse l'albumine de sérum humain (ASH).

4. Procédé selon la revendication 2, caractérisé en ce que l'on utilise comme source de sucre le colostrum de vache.

5. Procédé selon la revendication 1, caractérisé en ce que l'on utilise comme réactif de couplage le 3-(2-pyridyldithio)propionate de N-succinimidyle (SPDP).

6. Procédé pour la fabrication d'un vaccin, caractérisé en ce que l'on mélange des néoglycoprotéines, pouvant être obtenues selon la revendication 1, avec des véhicules et stabilisants du commerce.

7. Procédé pour la fabrication d'un agent de diagnostic, caractérisé en ce que l'on utilise des néoglycoprotéines pouvant être obtenues selon la revendication 1.
